# EUROPEAN PATENT SPECIFICATION

(11) **EP 3 821 926 B1**
(45) Date of publication and mention of the grant of the patent: **20.12.2023**
(21) Application number: 19833581.2
(22) Date of filing: 08.07.2019
(51) Int. Cl.: A61M 5/32, A61B 17/34

(54) **INJECTION INSTRUMENT SET**
INJEKTIONSBESTECK
ENSEMBLE INSTRUMENT D'INJECTION

(30) Priority: 09.07.2018 JP 2018129914
(43) Date of publication of application: 19.05.2021
(73) Proprietor: Keio University, Tokyo 108-8345 (JP); Heartseed Inc., Tokyo 106-0045 (JP)
(72) Inventor: KANAZAWA, Hideaki, Tokyo 160-8582 (JP); FUKUDA, Keiichi, Tokyo 160-8582 (JP); FUJITA, Jun, Tokyo 160-8582 (JP)
(74) Representative: V.O.
(86) International application number: PCT/JP2019/026979
(87) International publication number: WO 2020/013125

(56) References cited:
- WO-A2-2009/131774
- JP-A- S57 139 358
- JP-A- 2006 527 631
- JP-A- 2011 518 607
- JP-A- 2014 521 443
- JP-A- 2016 005 543
- JP-A- 2016 005 543

## Description

### [Technical Field]

The present invention relates to an injection instrument set.

Priority is claimed on Japanese Patent Application No. 2018-129914, filed July 9, 2018.

### [Background Art]

In recent years, regenerative medicine in which cells or aggregates thereof obtained by inducing differentiation from stem cells such as iPS cells are transplanted into an affected tissue has attracted attention.

Conventionally, in regenerative medicine, cells, drugs, and the like are injected into the affected tissue using an injection instrument. As an injection instrument, an injection instrument having an injection needle is known (refer to, for example, Patent Literature 1 and 2). The injection needle is formed in a tubular shape and is inserted into an injection target such as an affected tissue. Thus, the injection instrument injects an injection liquid containing cells, drugs, and the like into the injection target.

### [Citation List]

### [Patent Literature]

[Patent Literature 1]
   Published Japanese Translation No. 2013-514114 of the PCT International Publication
[Patent Literature 2]
   Published Japanese Translation No. 2013-544600 of the PCT International Publication
[Patent Literature 3]
   JP 2016 005543 A (i.e. Japanese Unexamined Patent Application, First Publication No. 2016-5543). It is noted that JP 2016 005543 A discloses an injection instrument set according to the pre-characterizing portion of the appended independent claim 1.
[Patent Literature 4]
   JP 2016 005543 A discloses an injection system that forms the basis of the preamble of claim 1.

### [Summary of Invention]

Claim 1 defines the invention and dependent claims define embodiments. No surgical methods are claimed.

### [Technical Problem]

However, the injection instruments described in Patent Literature 1 and 2 have a constitution in which an injection needle is inserted perpendicularly into a shallow portion. Therefore, the injection needle may pass beyond a shallow portion when the injection liquid needs to be injected into a shallow portion of an injection target, and the injection liquid may not be injected into this shallow portion.

Therefore, it is conceivable to insert the injection needle at an angle into the shallow portion. However, an operation in which the injection needle is inserted at an angle is unstable. Therefore, in the conventional injection instrument, it has been difficult to inject the injection liquid into a shallow portion. Further, in the injection needle and injection instrument set described in Patent Literature 3, since a syringe is disposed in an oblique direction, it is necessary to inject the injection liquid in the oblique direction, and since an inclined surface of a support block has a constant angle, it is difficult to make the injection needle correspond to the shallow portion according to a thickness of the tissue (the injection target).

An aspect of the present invention is in view of the above circumstances and is to provide an injection instrument set in which, when an injection liquid is injected into a shallow portion of an injection target, a syringe is not disposed in an oblique direction, a nearby side can be easily seen, an injection angle is easily changed according to a thickness of a tissue, and thus drugs or cells can be injected over a wide area and shallowly without passing beyond the tissue.

### [Solution to Problem]

In order to solve the problems and achieve the object, the present invention provides an injection instrument set according to the appended independent claim 1. Preferable embodiments of the present invention are provided by the appended dependent claims 2-7.

Accordingly, the present disclosure discloses the following aspects to aid understanding of the invention.
(1) An injection instrument set includes: a tubular injection instrument in which an injection liquid is accommodated; and a guide member disposed on a surface of an injection target of the injection liquid, wherein the injection instrument has an injection needle which is inserted into the injection target and injects the injection liquid into the injection target, and the guide member includes: a guide passage which is disposed at a predetermined angle with respect to a surface of the guide member in contact with the injection target, allows the injection needle to be inserted, and guides the injection needle to the surface of the injection target; and an anti-slip portion disposed at a portion at which the guide member comes into contact with the surface of the injection target.
   According to the aspect (1), the injection instrument having the injection needle and the guide member having the guide passage disposed obliquely with respect to the surface of the injection target are provided, and when the injection liquid is injected into a shallow portion of the injection target, an inclination angle of the guide passage is reduced, and the injection needle is inserted into the guide passage and inserted obliquely into the shallow portion. Further, the injection needle is guided by the guide passage and is obliquely inserted into the shallow portion by mounting the guide member on the surface (a tissue surface) of the injection target. Therefore, an insertion operation of the injection needle can be performed stably. In addition, the connection portion allows the syringe to be held in a state close to upright rather than in an oblique direction, and thus a field of view at the nearby side is widened. Therefore, the injection needle can be easily disposed in the shallow portion. Thus, in the above-described aspect (1), the injection liquid can be easily injected into the shallow portion of the injection target.
(2) In the aspect (1), the guide member may be formed of a transparent material.
   According to the aspect (2), since a state of the injection target can be visually observed, it is possible to perform an injection operation of the injection liquid while the state of the injection target is easily grasped. Thus, in the above-described aspect (2), the injection operation of the injection liquid can be efficiently performed.
(3) In the aspect (1) or (2), the guide member may have a positioning portion which comes into contact with the injection instrument when the injection needle is inserted and disposes an injection position of the injection liquid due to the injection needle in a shallow portion of the injection target.
   According to the aspect (3), when the injection needle is inserted, an injection position is reliably placed in the shallow portion of the injection target. Therefore, in the above-described aspect (3), the injection liquid can be more easily injected into the shallow portion of the injection target.
(4) In any one of the aspects (1) to (3), the injection instrument may have a plurality of injection needles, and the guide member may have a plurality of guide passages corresponding to the plurality of injection needles.
   According to the aspect (4), it is possible to inject a large amount of the injection liquid over a wide range as compared with a case in which one injection needle and one guide passage are provided. Therefore, in the above-described aspect (4), the injection liquid can be efficiently injected into the shallow portion of the injection target.
(5) In any one of the aspects (1) to (4), the guide passage may allow the injection needle to be inserted from both sides of the guide member in a predetermined first direction and may extend in the first direction, and one guide passage and the other guide passage of the guide passages on the both sides may be alternately disposed in a second direction intersecting the first direction.
   According to the aspect (5), it is possible to shorten a length of the guide member in the first direction as compared with a case in which the guide passages on both sides are disposed to face each other. Therefore, in the above-described aspect (5), it is possible to reduce a size of the guide member.
(6) In any one of the aspects (1) to (5), a tip end of the injection needle may be closed, and a plurality of discharge holes spirally disposed in a lengthwise direction of the injection needle may be disposed in a peripheral surface of a portion of the injection needle which is disposed in a shallow portion of the injection target.
   According to the aspect (6), when the injection liquid is injected, the injection liquid is spirally discharged from a plurality of discharge holes into the shallow portion, and thus the injection liquid can be injected into a wide range of the shallow portion. Therefore, in the above-described aspect (6), the injection liquid can be efficiently injected into the shallow portion of the injection target.
(7) In any one of the aspects (1) to (6), a diameter of an insertion side end portion of the injection needle of the guide passage may expand from an inner side to an outer side of the insertion side end portion in an axial direction.
   According to the aspect (7), an insertion range of the injection needle of the guide passage expands. Therefore, in the above-described aspect (7), the injection needle can be easily inserted into the guide passage.
(8) In any one of the aspects (1) to (7), the guide member may be fixed to the surface of the injection target and may be mounted in a state in which the guide member is able to be disposed between a vibration suppression device configured to curb vibration of the injection target and the surface of the injection target.
   According to the aspect (8), when the vibration suppression device is fixed to and disposed on the surface of the injection target, the guide member is disposed in a state in which it is fixed to the surface of the injection target. Therefore, it is not necessary to press the guide member so that the guide member does not move when the injection needle is inserted and when the injection liquid is injected. Thus, in the above-described aspect (8), the insertion operation of the injection needle and the injection operation of the injection liquid can be efficiently performed.
(9) In any one of the aspects (1) to (8), the injection instrument may include the injection needle, a mounting portion on which the injection needle is mounted, and an injection instrument main body which is connected to the mounting portion via a connection portion and delivers the injection liquid to the injection needle via the connection portion and the mounting portion, and the connection portion may be bent.

According to the aspect (9), when the injection needle is inserted, the connection portion can be disposed to be bent upward with respect to the surface of the injection target, and the insertion operation can be performed by holding the injection instrument main body. At this time, since the injection instrument main body is more upright than when the connection portion is not bent, the nearby side is visible and the injection instrument main body is sufficiently separated from the injection target and surroundings thereof. Therefore, the hand holding the injection instrument main body coming into contact with the injection target and the surroundings thereof is curbed, safety is ensured even during use in surgery, and the injection needle can be easily inserted.

### [Advantageous Effects of Invention]

According to the aspects of the present invention, an injection liquid can be easily injected into a shallow portion of an injection target by a simple operation.

### [Brief Description of Drawings]

FIG. 1 is a diagram showing a situation when an injection instrument set according to an embodiment of the present invention is used.
FIG. 2 is a cross-sectional view along line A-A of a mounting portion of FIG. 1.
FIG. 3 is a side view of an injection needle of FIG. 1.
FIG. 4 is a cross-sectional view along line B-B of FIG. 3.
FIG. 5 is a plan view of a guide member of FIG. 1.
FIG. 6 is a cross-sectional view along line C-C of FIG. 5.
FIG. 7 is an enlarged view of a portion D in FIG. 6.

### [Description of Embodiments]

Hereinafter, an embodiment of the present invention will be described with reference to the drawings.

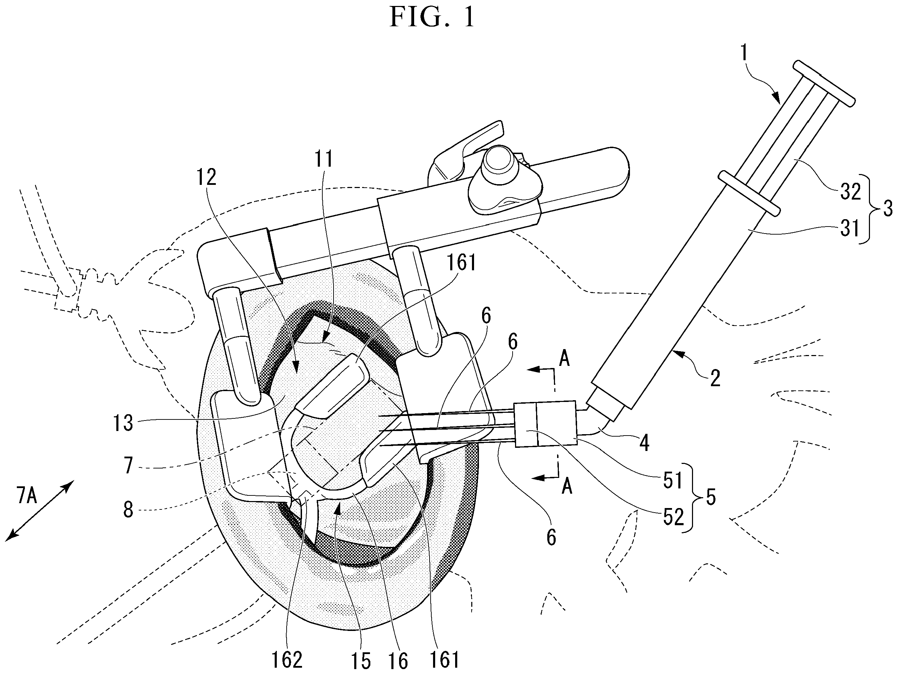

FIG. 1 is a diagram showing a situation when an injection instrument set according to an embodiment of the present invention is used.

The injection instrument set 1 of the embodiment is used for transplanting regenerated myocardial cells into a myocardium (a shallow portion) 12 of a heart (an injection target) 11. The regenerated myocardial cells are derived from, for example, iPS cells. The myocardium 12 is a portion formed on the surface side of the heart 11.

The injection instrument set 1 transplants the regenerated myocardial cells into the myocardium 12 by injecting a liquid (an injection liquid) containing the regenerated myocardial cells into the myocardium 12. The injection instrument set 1 includes an injection instrument 2, a guide member 7, and an auxiliary member 8.

The injection instrument 2 is an instrument for injecting an injection liquid into the myocardium 12. The injection instrument 2 includes an injection instrument main body 3, a connection portion 4, a mounting portion 5, and a plurality of (three) injection needles 6.

The injection instrument main body 3 includes a syringe 31 and a plunger 32.

The syringe 31 is formed in a tubular shape. The plunger 32 is fitted inside the syringe 31 from the base end side of the syringe 31. The plunger 32 is formed inside the syringe 31 to be movable in an axial direction of the syringe 31. An injection liquid is accommodated inside the syringe 31 between the syringe 31 and the plunger 32. The injection liquid is discharged from a tip end of the syringe 31 by the plunger 32 being manually pressed and moved to the tip end side inside the syringe 31.

The connection portion 4 connects the syringe 31 with the mounting portion 5. The connection portion 4 is formed in a tubular shape. The connection portion 4 is formed to be bent. A base end of the connection portion 4 is connected to a tip end of the syringe 31 by a Luer taper method. The inside of the connection portion 4 communicates with the inside of the syringe 31. The inside of the connection portion 4 is formed so that the injection liquid discharged from the syringe 31 can pass therethrough.

The mounting portion 5 is a portion on which the plurality of injection needles 6 are mounted. The mounting portion 5 includes a base portion 51 and a mounting portion main body 52.

The base portion 51 is formed in a tubular shape. A base end of the base portion 51 is coupled to a tip end of the connection portion 4. The base end of the base portion 51 is screwed into the tip end of the connection portion 4 via an O-ring (not shown). The inside of the base portion 51 allows the injection liquid delivered from the connection portion 4 to pass therethrough.

The mounting portion main body 52 is formed to protrude from the tip end side of the base portion 51. The mounting portion main body 52 is formed in a rectangular parallelepiped shape with a radial direction of the base portion 51 as a longitudinal direction. The base end side of the plurality of injection needles 6 is mounted on the mounting portion main body 52. The inside of each of the injection needles 6 communicates with the inside of the base portion 51. Accordingly, the injection liquid passing through the inside of the base portion 51 can be delivered to the inside of each of the injection needles 6.

The plurality of injection needles 6 are mounted in parallel on the mounting portion main body 52. The plurality of injection needles 6 are disposed to be spaced apart from each other at substantially equal intervals. The base end side of each of the injection needles 6 is mounted on the mounting portion main body 52. Each of the injection needles 6 is formed in a tubular shape. The inside of each of the injection needles 6 is formed so that the injection liquid delivered from the mounting portion 5 can pass therethrough. Each of the injection needles 6 is formed to be inserted into the heart 11 (inside the heart 11). Each of the injection needles 6 is formed so that the injection liquid inside the injection needle 6 can be injected into the heart 11 in a state in which it is inserted into the heart 11.

The guide member 7 is a member for assisting an injection operation of the injection liquid by the injection instrument 2. The guide member 7 is disposed on the surface 13 of the heart 11 (the myocardium 12). The guide member 7 is subjected to an anti-slip treatment on a portion thereof which comes into contact with the surface 13 of the heart 11. The guide member 7 is detachably mounted on a stabilizer (a vibration suppression device) 15.

The stabilizer 15 is fixed to and disposed on the surface 13 of the heart 11. The stabilizer 15 includes a U-shaped adsorption portion 16. The adsorption portion 16 is adsorbed and fixed to the surface 13 of the myocardium 12. Thus, the stabilizer 15 curbs vibration of the heart 11.

The guide member 7 is formed in a plate shape of a transparent material such as an acrylic resin. Most of the guide member 7 is detachably mounted between U-shaped side portions 161 and 161 of the adsorption portion 16 of the stabilizer 15. A part of the guide member 7 near an outer periphery thereof is disposed on a root portion 162 of the adsorption portion 16 of the stabilizer 15. In addition, "transparent" may mean light transmission to an extent that, for example, blood vessels and the like of the heart 11 can be visually observed through the material.

The auxiliary member 8 is formed in a plate shape. The auxiliary member 8 is disposed below the root portion 162 of the adsorption portion 16 of the stabilizer 15. The auxiliary member 8 is coupled to one end of the guide member 7. In this state, the guide member 7 and the auxiliary member 8 detachably sandwich the root portion 162 of the adsorption portion 16. Thus, the guide member 7 is detachably mounted on the stabilizer 15 using the auxiliary member 8.

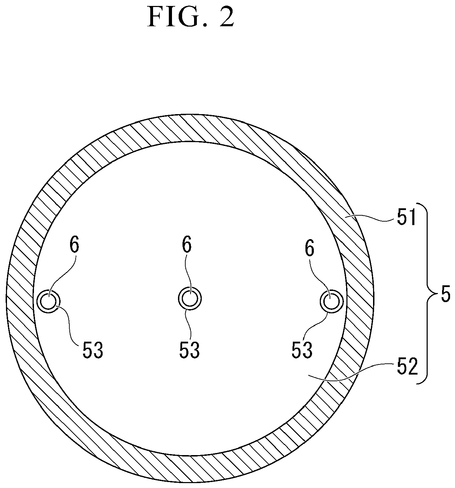

FIG. 2 is a cross-sectional view along line A-A of the mounting portion of FIG. 1.

As shown in FIG. 2, a plurality of (three) mounting holes 53 are provided inside the mounting portion 5. The plurality of mounting holes 53 are formed to linearly pass through the inside of the mounting portion 5 (the inside of the base portion 51 and the mounting portion main body 52) from the base end side to the tip end side of the mounting portion 5. The plurality of mounting holes 53 are disposed in parallel in a radial direction of the mounting portion main body 52. The tip end side of each of the injection needles 6 is fitted into each of the mounting holes 53. Accordingly, the plurality of injection needles 6 are mounted on the mounting portion main body 52.

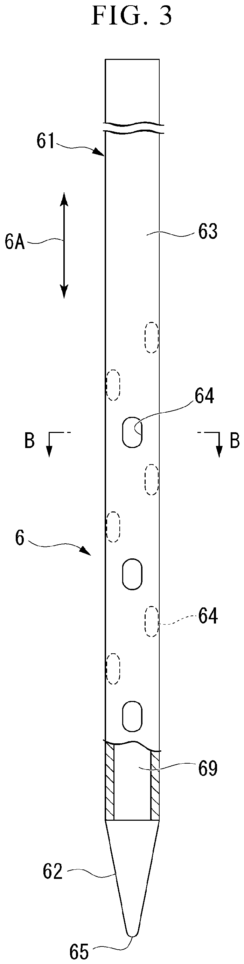

FIG. 3 is a side view of the injection needle of FIG. 1. In addition, in FIG. 3, a part of the tip end side of the injection needle 6 is shown in a cross-sectional view.

As shown in FIG. 3, the injection needle 6 includes an injection needle main body 61 and a tip end portion 62. The injection needle main body 61 is formed in a tubular shape. A plurality of discharge holes 64 are provided in a peripheral surface 63 of the injection needle main body 61. A portion of the injection needle main body 61 in which the discharge hole 64 is formed is a portion to be disposed in the myocardium 12 when the injection needle 6 is inserted. The plurality of discharge holes 64 are spirally provided in the peripheral surface 63 in a lengthwise direction 6A (an axial direction) of the injection needle 6. The plurality of discharge holes 64 are formed to discharge the injection liquid accommodated in the inside 69 of the injection needle main body 61 to the myocardium 12.

The tip end portion 62 is provided on the tip side of the injection needle main body 61. The tip end portion 62 forms a tip end portion of the injection needle 6. The tip end portion 62 is formed to be tapered from a base end to a tip end of the tip end portion 62. A tip end 65 of the injection needle 6 is a closed blind end.

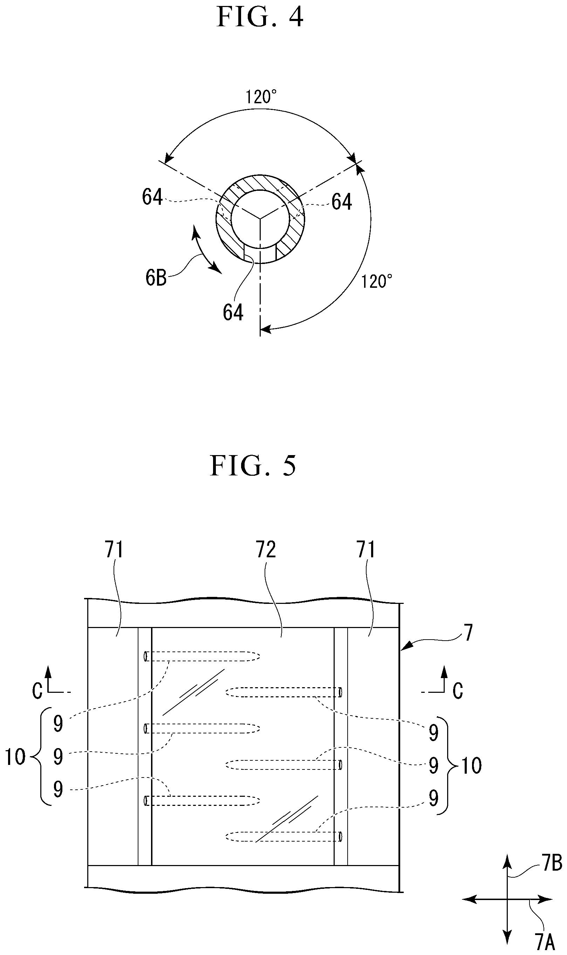

FIG. 4 is a cross-sectional view along line B-B of FIG. 3.

As shown in FIG. 4, the plurality of discharge holes 64 are disposed at equal intervals in a circumferential direction 6B of the injection needle 6 when seen in the length direction 6A of the injection needle 6. Specifically, the discharge holes 64 are disposed at intervals of 120 ° in the circumferential direction 6B of the injection needle 6.

FIG. 5 is a plan view of the guide member of FIG. 1. The guide member 7 of FIGS. 1 and 5 shows a part of an upper portion of the guide member 7 in which the plurality of mounting holes 53 are disposed. A planar shape of the guide member 7 is, for example, a circle or a rectangle.

As shown in FIG. 5, recessed portions 71 are provided in both side portions of the guide member 7 in a predetermined first direction 7A (a left-right direction in FIG. 5). Both recessed portions 71 and 71 are formed by cutting out upper surface side portions of both side portions. Guide passage groups 10 are respectively provided on both sides of the guide member 7 in the first direction 7A. The guide passage groups 10 and 10 on both sides are provided on both sides of an intermediate portion 72 in the first direction 7A which is a portion (the intermediate portion 72) between the recessed portions 71 and 71 of the guide member 7.

Each of the guide passage groups 10 is constituted of a plurality of (three) guide passages 9. Each of the plurality of guide passages 9 corresponds to each of the plurality of (three) injection needles 6. Each of the plurality of guide passages 9 on both sides is provided so that the injection needle 6 can be inserted therein. The plurality of guide passages 9 on both sides are provided to extend in the first direction 7A. The plurality of guide passages 9 are alternately disposed in a second direction 7B (a vertical direction in FIG. 5) intersecting the first direction 7A.

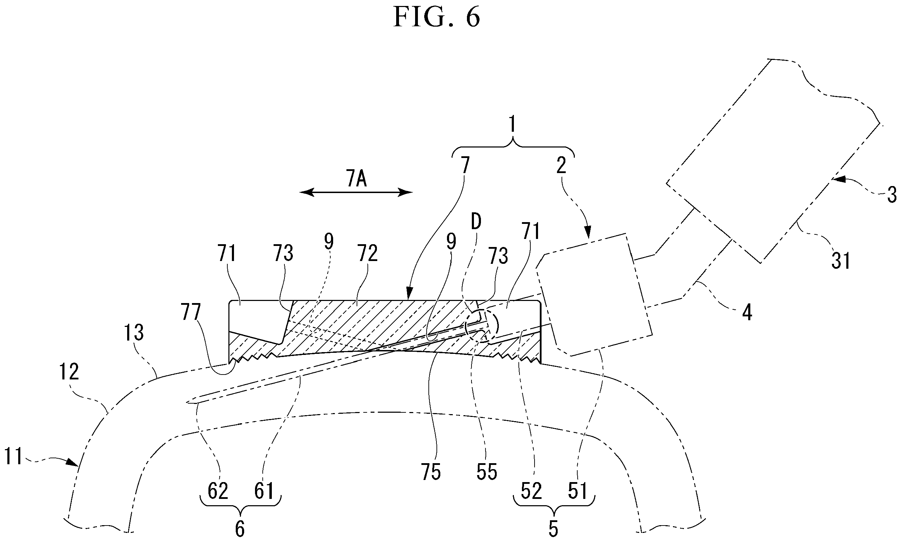

FIG. 6 is a cross-sectional view along line C-C of FIG. 5. In FIG. 6, the heart 11 and the injection instrument 2 are illustrated by a virtual line (an alternating two dots-dashed line).

As shown in FIG. 6, each of the guide passages 9 is disposed obliquely with respect to the surface 13 of the myocardium 12. Each of the guide passages 9 is provided to linearly pass through the inside of the intermediate portion 72 from a side surface 73 of the intermediate portion 72 of the guide member 7 in the first direction 7A (an inner surface of the recessed portion 71 in the first direction 7A) toward a bottom surface 75 of the guide member 7. The bottom surface 75 of the guide member 7 is a surface which comes into contact with the surface 13 of the myocardium 12 when the guide member 7 is disposed on the surface 13 of the myocardium 12. Each of the guide passages 9 is formed so that the injection needle 6 can be inserted from the side surface 73 side of the intermediate portion 72 and can be guided to the surface 13 of the myocardium 12. An inclination angle of the guide passage 9 may be changed according to a thickness of the myocardium 12.

A plurality of grooves 77 are provided on an outer peripheral edge portion of the bottom surface 75 of the guide member 7 as an anti-slip treatment (an anti-slip portion) which prevents the guide member 7 from being shifted on the surface 13. The plurality of grooves 77 are formed to be arranged and disposed on the entire edge portion of the bottom surface 75. The plurality of grooves 77 may be formed linearly or in waves. Further, the anti-slip portion may be a plurality of point-shaped protrusions other than the grooves.

The mounting portion main body 52 of the injection instrument 2 is formed to be disposed in the recessed portion 71 when the injection needle 6 is inserted. A tip end surface 55 of the mounting portion main body 52 is formed to come into contact with the side surface 73 of the intermediate portion 72 of the guide member 7 when an injection position of the injection needle 6 is disposed at a predetermined portion of the myocardium 12. The tip end surface 55 of the mounting portion main body 52 and the side surface 73 of the intermediate portion 72 correspond to positioning portions of the embodiment. The injection position of the injection liquid with respect to the injection needle 6 is a portion of the injection needle main body 61 on the tip end side and is a portion of the injection needle 6 in which the plurality of discharge holes 64 are provided. The predetermined portion of the myocardium 12 is, for example, an intermediate portion of the myocardium 12 in a thickness direction. A length of the injection needle 6 is set to a length which reaches the intermediate portion of the myocardium 12 in the thickness direction when the injection needle 6 is inserted diagonally.

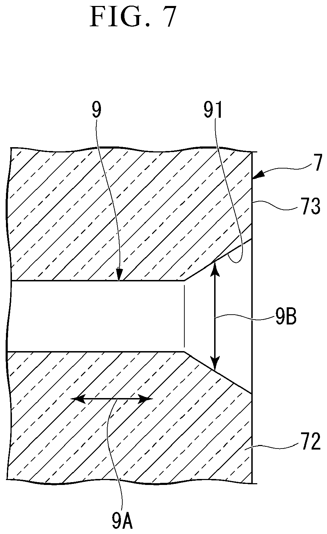

FIG. 7 is an enlarged view of a portion D in FIG. 6.

As shown in Fig. 7, an insertion side end portion 91 of the injection needle 6 of the guide passage 9 is formed by expanding a diameter thereof from the inner side to the outer side of the insertion side end portion 91 in an axial direction 9A (an axial direction of the guide passage 9). In other words, a diameter 9B of the insertion side end portion 91 is formed to increase from the inner side to the outer side of the insertion side end portion 91 in the axial direction 9A. The insertion side end portion 91 is an end portion of both end portions (opening end portions on both sides) of the guide passage 9 on the side on which the injection needle 6 is inserted.

Next, a method of injecting an injection liquid using the injection instrument set 1 will be described. The injection liquid is accommodated inside the syringe 31 of the injection instrument main body 3.

First, a user places the stabilizer 15 on the surface 13 of the heart 11 (that is, the surface 13 of the myocardium 12). Thus, the guide member 7 is disposed on the surface 13 of the heart 11 together with the stabilizer 15.

Then, the user disposes the connection portion 4 to be bent upward with respect to the surface 13 of the heart 11 and holds the injection instrument main body 3. Next, the user inserts the plurality of injection needles 6 into the plurality of guide passages 9 of the guide member 7 and obliquely inserts the plurality of injection needles into the inside of the myocardium 12.

Next, the user brings the tip end surface 55 of the mounting portion main body 52 of the injection instrument 2 into contact with the side surface 73 of the intermediate portion 72 of the guide member 7. Thus, the injection position of the injection liquid with respect to the injection needle 6 is disposed at a predetermined portion of the myocardium 12.

Next, the user presses the plunger 32 of the injection instrument main body 3 and moves it to the tip end side inside the syringe 31. Thus, the injection liquid is discharged from the tip end of the syringe 31. The injection liquid discharged from the tip end of the syringe 31 is delivered to the inside 69 of each of the injection needles 6 through the inside of the connection portion 4 and the mounting portion 5. The injection liquid delivered to the inside 69 of each of the injection needles 6 is discharged to the myocardium 12 from the plurality of discharge holes 64. Accordingly, the injection liquid is injected into the myocardium 12.

Next, an action and an effect of the injection instrument set 1 of the embodiment will be described.

The injection instrument set 1 of the embodiment includes the injection instrument 2 formed in a tubular shape and including an injection needle 6 which is inserted into the heart 11 as an injection target and injects an injection liquid, and the guide member 7 disposed on the surface 13 of the heart 11. The guide passage 9 disposed obliquely with respect to the surface 13 of the heart 11 is provided in the guide member 7. The guide passage 9 is formed to allow the injection needle 6 to be inserted and guided to the surface 13 of the heart 11.

Thus, when the injection liquid is injected into the myocardium 12 which is a shallow portion of the heart 11, the injection needle 6 is inserted into the guide passage 9 and inserted obliquely into the myocardium 12. Further, the injection needle 6 is guided by the guide passage 9 and is obliquely inserted into the myocardium 12 by mounting the guide member 7 on the surface 13 of the heart 11. Therefore, an insertion operation of the injection needle 6 can be stably performed. Therefore, the injection needle 6 can be easily disposed in the myocardium 12. Therefore, the injection instrument set 1 of the embodiment can easily inject the injection liquid into the myocardium 12. Further, since the injection needle 6 can be easily disposed in the myocardium 12, it is possible to prevent the injection needle 6 from being inserted into the myocardium 12.

In the injection instrument set 1 of the embodiment, the guide member 7 is formed of a transparent material.

Thus, since a state of the heart 11 can be visually observed, it is possible to inject the injection liquid while the state of the heart 11 is easily grasped. Specifically, for example, since the blood vessels or the like of the heart 11 can be visually observed through the transparent guide member 7, it is possible to perform the injection operation of the injection liquid while the blood vessels are avoided. Therefore, the injection instrument set 1 of the embodiment can efficiently perform the injection operation of the injection liquid. In addition, when a problem such as bleeding occurs during the injection operation, it can be immediately recognized and can be dealt with promptly.

In the injection instrument set 1 of the embodiment, the positioning portions (the tip end surface 55 of the mounting portion main body 52 of the injection instrument 2 and the side surface 73 of the intermediate portion 72 of the guide member 7) which are in contact with each other when the injection needle 6 is inserted and dispose the injection position of the injection needle 6 in the myocardium 12 are provided on the injection instrument 2 and the guide member 7. Thus, when the injection needle 6 is inserted, the injection position is reliably disposed in the myocardium 12. Therefore, the injection instrument set 1 of the embodiment can more easily inject the injection liquid into the myocardium 12.

In the injection instrument set 1 of the embodiment, the injection instrument 2 includes the plurality of injection needles 6, and the guide member 7 has the plurality of guide passages 9 corresponding to the plurality of injection needles 6.

Thus, when compared with a case in which one injection needle 6 and one guide passage 9 are provided, it is possible to inject a large amount of the injection liquid over a wide area. Therefore, the injection instrument set 1 of the embodiment can efficiently inject the injection liquid into the myocardium 12.

In the injection instrument set 1 of the embodiment, the guide passage 9 extends in the first direction 7A and is provided so that the injection needles 6 can be inserted from both sides of the guide member 7 in the predetermined first direction 7A. The guide passages 9 on both sides are alternately disposed in the second direction 7B intersecting the first direction 7A. Thus, the length of the guide member 7 in the first direction 7A can be shortened as compared with the case in which the guide passages 9 on both sides are disposed to face each other. Therefore, the injection instrument set 1 of the embodiment can have a reduced size for the guide member 7.

Further, in the injection instrument set 1 of the embodiment, since the guide passages 9 are provided on both sides of the guide member 7 in the first direction 7A, the insertion position of the injection needle 6 can be determined to be one side or the other side in the first direction 7A according to the state of the heart 11 or the like. Therefore, the injection instrument set 1 of the embodiment can improve usability.

In the injection instrument set 1 of the embodiment, the tip end 65 of the injection needle 6 is formed to be closed.

In the injection needle 6, the plurality of discharge holes 64 are spirally provided in the peripheral surface 63 of the portion disposed on the myocardium 12 in the lengthwise direction 6A of the injection needle 6 when the injection needle 6 is inserted. Therefore, when the injection liquid is injected, since the injection liquid is discharged into the myocardium 12 from the plurality of discharge holes 64, the injection liquid can be injected into a wide range of the myocardium 12. Thus, the injection instrument set 1 of the embodiment can efficiently inject the injection liquid into the myocardium 12.

In the injection instrument set 1 of the embodiment, the insertion side end portion 91 of the injection needle 6 of the guide passage 9 is formed by expanding the diameter from the inner side to the outer side of the insertion side end portion 91 in the axial direction 9A. Thus, the insertion range of the injection needle 6 of the guide passage 9 expands. Therefore, in the injection instrument set 1 of the embodiment, the injection needle 6 can be easily inserted into the guide passage 9.

In the injection instrument set 1 of the embodiment, the guide member 7 is fixed to and disposed on the surface 13 of the heart 11 and is mounted on the stabilizer 15 (the vibration suppression device) which curbs the vibration of the heart 11 in a state in which it is able to be disposed on the surface 13 of the heart 11. Therefore, the guide member 7 is disposed on the surface 13 of the heart 11 together with the stabilizer 15 when the stabilizer 15 is disposed on the surface 13 of the heart 11. Therefore, the user does not need to press the guide member 7 so that the guide member 7 does not move when the injection needle 6 is inserted and when the injection liquid is injected. Thus, the injection instrument set 1 of the embodiment can efficiently perform the insertion operation of the injection needle 6 and the injection operation of the injection liquid.

Further, in the injection instrument set 1 of the embodiment, the guide member 7 is detachably mounted on the stabilizer 15. Therefore, during maintenance of the guide member 7 and the stabilizer 15, maintenance can be performed in a state in which both are separated by removing the guide member 7 from the stabilizer 15. Thus, the injection instrument set 1 of the embodiment can improve workability of the maintenance operation of the guide member 7 and the stabilizer 15.

In the injection instrument set 1 of the embodiment, the injection instrument 2 includes the injection needle 6 and the mounting portion 5 on which the injection needle 6 is mounted. Further, the injection instrument 2 is connected to the mounting portion 5 via the connection portion 4 and includes the injection instrument main body 3 which delivers the injection liquid to the injection needle 6 via the connection portion 4 and the mounting portion 5. The connection portion 4 is formed to be bent. Therefore, when the injection needle 6 is inserted, the connection portion 4 is disposed to be bent upward with respect to the surface 13 of the heart 11, and the insertion operation is performed by holding the injection instrument main body. At this time, since the injection instrument main body 3 stands up more than when the connection portion 4 is not bent, the nearby side is visible, and the injection instrument main body 3 is sufficiently separated from the heart 11 and surroundings thereof. Thus, the hand holding the injection instrument main body 3 coming into contact with the heart 11 and the surroundings thereof is curbed, safety is ensured even during use in surgery, and the insertion operation of the injection needle 6 can be easily performed.

In the injection instrument set 1 of the embodiment, the groove 77 is provided in the bottom surface 75 of the guide member 7. Therefore, when the guide member 7 is disposed on the surface 13 of the myocardium 12, it is possible to curb slipping of the guide member 7 on the surface 13 of the myocardium 12. Therefore, since the guide member 7 is stably disposed on the surface 13 of the myocardium 12, it is possible to stably perform the insertion operation of the injection needle 6 and the injection operation of the injection liquid. Thus, the injection instrument set 1 of the embodiment can efficiently perform the insertion operation of the injection needle 6 and the injection operation of the injection liquid.

Although embodiments have been described in detail with reference to the drawings, the specific constitution is not limited to the embodiments shown.

In the injection instrument set 1 of the embodiment, although the insertion side end portion 91 of the guide passage 9 is formed by expanding the diameter thereof, the insertion side end portion 91 may be formed by increasing the diameter using a separate member.

Specifically, for example, a member having a tubular fitting portion in the guide passage 9 can be used as the separate member. The fitting portion is detachably fitted to the insertion side end portion 91 of the guide passage 9 and is formed so that the injection needle 6 can be inserted therein. The insertion side end portion of the injection needle 6 of the fitting portion is formed by expanding a diameter thereof. Therefore, it is possible to easily insert the injection needle 6 into the fitting portion. Thus, the injection needle 6 can be easily inserted into the guide passage 9 by inserting the injection needle 6 into the guide passage 9 via the fitting portion.

In the injection instrument set 1 of the embodiment, although the guide member 7 is mounted on the stabilizer 15, the guide member 7 does not necessarily have to be mounted on the stabilizer 15.

In addition, for example, a grip portion which can be gripped by the user may be provided on the upper surface of the guide member 7. In this case, the guide member 7 is disposed on the surface 13 of the heart 11, and the guide member 7 is pressed by gripping the grip portion in this state. Thus, since it is possible to curb movement of the guide member 7, the insertion operation of the injection needle 6 and the injection operation of the injection liquid can be efficiently performed.

In addition, it is possible to replace components in the above-described embodiment with well-known components as appropriate without departing from the scope of the disclosure. The scope of the invention is solely defined by the appended claims.

### [Reference Signs List]

1 Injection instrument set
2 Injection instrument
3 Injection instrument main body
4 Connection portion
5 Mounting portion
6 Injection needle
6A Lengthwise direction
7 Guide member
7A First direction
7B Second direction
9 Guide passage
9AAxial direction
11 Heart (injection target)
12 Myocardium (shallow portion)
13 Surface of heart (surface of injection target)
15 Stabilizer (vibration suppression device)
55 Tip end surface of mounting portion main body of injection instrument (positioning portion)
63 Peripheral surface
64 Discharge hole
65 Tip end
73 Side surface of intermediate portion of guide member (positioning portion)
91 Insertion side end portion

## Claims

1. An injection instrument set (1) comprising:
a tubular injection instrument (2) configured for accommodating an injection liquid,
wherein the injection instrument (2) has an injection needle (6) which is configured to be inserted into an injection target (11) and which is configured to inject the injection liquid into the injection target (11), and
wherein a plurality of discharge holes (64), which are spirally disposed in a lengthwise direction (6A) of the injection needle (6), are disposed in a peripheral surface (63) of a portion of the injection needle (6) which is configured to be disposed in a shallow portion (12) of the injection target (11),
**characterized by:**
a guide member (7) configured to be disposed on a surface (13) of the injection target (11) of the injection liquid, wherein the guide member (7) includes:
a guide passage (9) which is disposed at a predetermined angle with respect to a surface of the guide member (7) for coming in contact with the injection target (11), the guide passage (9) being configured to allow the injection needle (6) to be inserted into the guide passage (9) and to guide the injection needle (6) to the surface (13) of the injection target (11);
an anti-slip portion (77) disposed at a portion at which the guide member (7) comes into contact with the surface (13) of the injection target (11); and
a positioning portion (73) which is configured to come into contact with the injection instrument (2) when the injection needle (6) is inserted into the guide passage (9) and which is configured to dispose, in a shallow portion (12) of the injection target (11), an injection position of the injection liquid due to the injection needle (6).

2. The injection instrument set (1) according to claim 1, wherein the guide member (7) is formed of a transparent material.

3. The injection instrument set (1) according to claim 1 or 2, wherein
the injection instrument (2) has a plurality of injection needles (6), and
the guide member (7) has a plurality of guide passages (9) corresponding to the plurality of injection needles (6).

4. The injection instrument set (1) according to any one of claims 1 to 3, wherein
the guide passage (9) allows the injection needle (6) to be inserted from both sides of the guide member (7) in a predetermined first direction (7A) and extends in the first direction, and
one guide passage and the other guide passage of the guide passages on the both sides are alternately disposed in a second direction (7B) intersecting the first direction.

5. The injection instrument set (1) according to any one of claims 1 to 4, wherein a diameter (9B) of an insertion side end portion (91) of the injection needle (6) of the guide passage (9) expands from an inner side to an outer side of the insertion side end portion (91) in an axial direction (9A).

6. The injection instrument set (1) according to any one of claims 1 to 5, wherein the guide member (7) is fixed to the surface (13) of the injection target (11) and mounted in a state in which the guide member (7) is able to be disposed between a vibration suppression device (15) configured to curb vibration of the injection target (11) and the surface (13) of the injection target (11).

7. The injection instrument set (1) according to any one of claims 1 to 6, wherein the injection instrument (2) includes the injection needle (6), a mounting portion (5) on which the injection needle (6) is mounted, and an injection instrument main body (3) which is connected to the mounting portion (5) via a connection portion (4) and delivers the injection liquid to the injection needle (6) via the connection portion (4) and the mounting portion (5), and the connection portion (4) is bent.

## Patentansprüche

1. Injektionsbesteck (1), umfassend:
ein rohrförmiges Injektionsinstrument (2), das zum Aufnehmen einer Injektionsflüssigkeit konfiguriert ist,
wobei das Injektionsinstrument (2) eine Injektionsnadel (6) aufweist, die konfiguriert ist, um in ein Injektionsziel (11) eingeführt zu werden, und die konfiguriert ist, um die Injektionsflüssigkeit in das Injektionsziel (11) zu injizieren, und
wobei eine Vielzahl von Auslasslöchern (64), die spiralförmig in einer Längsrichtung (6A) der Injektionsnadel (6) angeordnet sind, in einer Umfangsfläche (63) eines Abschnitts der Injektionsnadel (6) angeordnet sind, der konfiguriert ist, um in einem flachen Abschnitt (12) des Injektionsziels (11) angeordnet zu werden,
**gekennzeichnet durch**:
ein Führungselement (7), das konfiguriert ist, um auf einer Oberfläche (13) des Injektionsziels (11) der Injektionsflüssigkeit angeordnet zu werden, wobei das Führungselement (7) Folgendes einschließt:
einen Führungskanal (9), der in einem vorbestimmten Winkel in Bezug auf eine Oberfläche des Führungselements (7) angeordnet ist, um mit dem Injektionsziel (11) in Kontakt zu kommen, wobei der Führungskanal (9) derart konfiguriert ist, dass er das Einführen der Injektionsnadel (6) in den Führungskanal (9) ermöglicht und die Injektionsnadel (6) zu der Oberfläche (13) des Injektionsziels (11) führt;
einen Anti-Rutsch-Abschnitt (77), der an einem Abschnitt angeordnet ist, an dem das Führungselement (7) in Kontakt mit der Oberfläche (13) des Injektionsziels (11) kommt; und
einen Positionierungsabschnitt (73), der konfiguriert ist, um mit dem Injektionsinstrument (2) in Kontakt zu kommen, wenn die Injektionsnadel (6) in den Führungskanal (9) eingeführt wird, und der konfiguriert ist, um in einem flachen Abschnitt (12) des Injektionsziels (11) eine Injektionsposition der Injektionsflüssigkeit mittels der Injektionsnadel (6) anzuordnen.

2. Injektionsbesteck (1) nach Anspruch 1, wobei das Führungselement (7) aus einem transparenten Material gebildet ist.

3. Injektionsbesteck (1) nach Anspruch 1 oder 2, wobei
das Injektionsinstrument (2) eine Vielzahl von Injektionsnadeln (6) aufweist, und
das Führungselement (7) eine Vielzahl von Führungskanälen (9) aufweist, die der Vielzahl von Injektionsnadeln (6) entsprechen.

4. Injektionsbesteck (1) nach einem der Ansprüche 1 bis 3,
wobei der Führungskanal (9) das Einführen der Injektionsnadel (6) von beiden Seiten des Führungselements (7) in einer vorbestimmten ersten Richtung (7A) ermöglicht und sich in der ersten Richtung erstreckt, und
der eine Führungskanal und der andere Führungskanal der Führungskanäle auf den beiden Seiten abwechselnd in einer zweiten Richtung (7B) angeordnet sind, welche die erste Richtung kreuzt.

5. Injektionsbesteck (1) nach einem der Ansprüche 1 bis 4, wobei sich ein Durchmesser (9B) eines einführseitigen Endabschnitts (91) der Injektionsnadel (6) des Führungskanals (9) von einer Innenseite zu einer Außenseite des einführseitigen Endabschnitts (91) in einer axialen Richtung (9A) erweitert.

6. Injektionsbesteck (1) nach einem der Ansprüche 1 bis 5, wobei das Führungselement (7) an der Oberfläche (13) des Injektionsziels (11) befestigt ist und in einem Zustand montiert ist, in dem das Führungselement (7) zwischen einer Vibrationsunterdrückungsvorrichtung (15), die konfiguriert ist, um Vibrationen des Injektionsziels (11) zu dämpfen, und der Oberfläche (13) des Injektionsziels (11) angeordnet werden kann.

7. Injektionsbesteck (1) nach einem der Ansprüche 1 bis 6, wobei das Injektionsbesteck (2) die Injektionsnadel (6), einen Montageabschnitt (5), an dem die Injektionsnadel (6) montiert ist, und einen Injektionsbesteck-Hauptkörper (3) einschließt, der über einen Verbindungsabschnitt (4) mit dem Montageabschnitt (5) verbunden ist und die Injektionsflüssigkeit über den Verbindungsabschnitt (4) und den Montageabschnitt (5) an die Injektionsnadel (6) abgibt, und der Verbindungsabschnitt (4) gebogen ist.

## Revendications

1. Ensemble formant instrument d'injection (1) comprenant :
un instrument d'injection tubulaire (2) configuré pour loger une liquide d'injection, dans lequel l'instrument d'injection (2) comporte une aiguille d'injection (6) qui est configurée pour être insérée dans une cible d'injection (11) et qui est configurée pour injecter le liquide d'injection dans la cible d'injection (11), et dans lequel une pluralité d trous d'évacuation (64), qui sont disposés en spirale dans le sens de la longueur (6A) de l'aiguille d'injection (6), sont disposés dans une surface périphérique (63) d'une partie de l'aiguille d'injection (6) qui est configurée pour être disposée dans une partie peu profonde (12) de la cible d'injection (11), **caractérisé par**:
un élément de guidage (7) configuré pour être disposé sur une surface (13) de la cible de l'injection (11) du liquide d'injection, dans lequel l'élément de guidage (7) comprend :
un passage de guidage (9) qui est disposé selon un angle prédéterminé par rapport à une surface de l'élément de guidage (7) pour venir en contact avec la cible d'injection (11), le passage de guidage (9) étant configuré pour permettre à l'aiguille d'injection (6)d'être insérée dans le passage de guidage (9) et à guider l'aiguille d'injection (6) vers la surface (13) de la cible d'injection (11) ;
une partie antidérapante (77) disposée au niveau d'une partie au niveau de laquelle l'élément de guidage (7) entre en contact avec la surface (13) de la cible d'injection (11) ; et
une partie de positionnement (73) qui est configurée pour entrer en contact avec l'instrument d'injection (2) lorsque l'aiguille d'injection (6) est insérée dans le passage de guidage (9) et qui est configuré pour disposer, dans une partie peu profonde (12) de la cible d'injection (11), une position d'injection du liquide d'injection due à l'aiguille d'injection (6).

2. Ensemble formant instrument d'injection (1) selon la revendication 1, dans lequel l'élément de guidage (7) est formé d'un matériau transparent.

3. Ensemble formant instrument d'injection (1) selon la revendication 1 ou 2, dans lequel l'instrument d'injection (2) a une pluralité d'aiguilles d'injection (6), et l'élément de guidage (7) a une pluralité de passages de guidage (9) correspondant à la pluralité d'aiguilles d'injection (6).

4. Ensemble formant instrument d'injection (1) selon l'une quelconque des revendications 1 à 3, dans lequel le passage de guidage (9) permet à l'aiguille d'injection (6) d'être insérée des deux côtés de l'élément de guidage (7) dans une première direction prédéterminée (7A) et s'étend dans la première direction, et un passage de guidage et l'autre passage de guidage des passages de guidage des deux côtés sont disposés en alternance dans une seconde direction (7B) coupant la première direction.

5. Ensemble formant instrument d'injection (1) selon l'une quelconque des revendications 1 à 4, dans lequel un diamètre (9B) d'une partie d'extrémité côté insertion (91) de l'aiguille d'injection (6) du passage de guidage (9) s'étend d'un côté interne vers un côté externe de la partie d'extrémité côté insertion (91) dans une direction axiale (9A).

6. Ensemble formant instrument d'injection (1) selon l'une quelconque des revendications 1 à 5, dans lequel l'élément de guidage (7) est fixé à la surface (13) de la cible d'injection (11) et monté dans un état dans lequel l'élément de guidage (7) peut être disposé entre un dispositif de suppression de vibration (15) configuré pour freiner les vibrations de la cible d'injection (11) et de la surface (13) de la cible d'injection (11).

7. Ensemble formant instrument d'injection (1) selon l'une quelconque des revendications 1 à 6, dans lequel l'instrument d'injection (2) comprend l'aiguille d'injection (6), une partie de montage (5) sur laquelle l'aiguille d'injection (6) est montée, et un corps principal d'instrument d'injection (3) qui est relié à la partie de montage (5).via une partie de connexion (4) et délivre le liquide d'injection à l'aiguille d'injection (6) via la partie de connexion (4) et la partie de montage (5), et la partie de connexion (4) est pliée.
